# EUROPEAN PATENT SPECIFICATION

(11) **EP 4 218 682 B1**
(45) Date of publication and mention of the grant of the patent: **12.08.2026**
(21) Application number: 22208138.2
(22) Date of filing: 18.12.2018
(51) Int. Cl.: A61F 2/28

(54) **FLEXIBLE POROUS IMPLANT FIXATION SYSTEM**
FLEXIBLES PORÖSES IMPLANTATFIXIERUNGSSYSTEM
SYSTÈME DE FIXATION D'IMPLANT POREUX FLEXIBLE

(30) Priority: 20.12.2017 EP 17306847
(43) Date of publication of application: 02.08.2023
(62) Document number(s) of the earlier application(s) in accordance with Art. 76 EPC: 18830352.3 / 3 727 202
(73) Proprietor: Materialise NV, 3001 Leuven (BE); OBL SAS, 92240 Malakoff (FR)
(72) Inventor: ADAM, Jérémy, Chatillon (FR); GEEBELEN, Benjamin, Leuven (BE)
(74) Representative: Abel & Imray LLP

(56) References cited:
- WO-A1-2017/042366
- US-A1- 2013 218 288

## Description

### CROSS-REFERENCE TO RELATED APPLICATIONS

This application claims the benefit of European Patent Application No. 17306847.9, filed December 20, 2017.

### BACKGROUND OF THE INVENTION

### Field of the Invention

The present disclosure relates to medical implants. More particularly, the present disclosure relates to flexible porous implant fixation systems.

### Description of the Related Technology

Porous or partly porous bone implants may be used in certain circumstances. They can be made porous for different reasons, such as to save weight, to decrease thermal conductivity, to achieve mechanical properties that come closer to those of the surrounding bone, and/or to allow bone ingrowth for better fixation.

When designing a bone implant to allow bone ingrowth for better fixation, implant designers face conflicting requirements: on the one hand the stiffness of the implant should be lower than that of the surrounding bone, and on the other hand the implant should be strong enough to survive accidental impacts.

In particular, implants that are stiffer than the surrounding bone can cause stress shielding. Stress shielding is the phenomenon in which the implant takes on most of the loading such that less of the loading is carried by the bone. This can lead to bone resorption. In other words, the opposite of bone ingrowth may occur. To lower the stiffness of a porous implant, one strategy is to lower the density of the porous structure.

However, a porous structure with a lower density often also has a reduced strength. This is detrimental, as it increases the risk of the implant breaking under accidental loads. To increase the strength of a porous implant, one strategy is to increase the density of its porous structure.

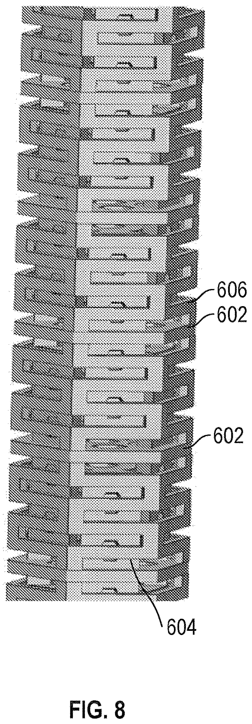

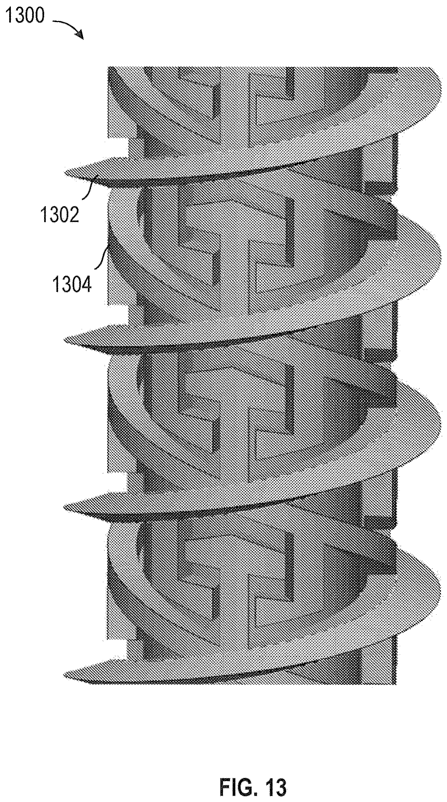

International patent application WO/2017/042366 discloses a porous structure that allows reconciling both requirements: a low stiffness under normal loading and a high stiffness under accidental loading, such as in the Abstract and Fig. 8b of WO/2017/042366. In some embodiment, this porous structure comprises units that are connected by means of connection elements, such as in Fig. 13c and described in the specification at p. 20, ll. 15-21 of WO/2017/042366. Each pair of adjacent units has two surfaces that make contact upon larger-than-normal deformation, such as described in the Summary section of the specification of WO/2017/042366. In this way, the connection elements can be dimensioned to deliver the low stiffness required to promote bone ingrowth when the surfaces do not touch, and the units can be designed to deliver, when their surfaces touch, the strength required to survive accidental loads, such as described in the Summary section of the specification of WO/2017/042366.

Implants and porous structures that provide further novel and non-obvious improvements to the implants and porous structures described in WO/2017/042366, such as described in the Summary section of the specification of WO/2017/042366, are further described herein.

### BRIEF DESCRIPTION OF THE DRAWINGS

FIG. 1 is an example of a porous structure having a body that is a helicoidal bar with integrated interlocking elements, according to certain aspects.
FIG. 2 is an example of a porous structure having a body that is a zig-zag shape with integrated interlocking elements, according to certain aspects.
FIG. 3 is an example of a porous structure having a body that is a perforated sheet with integrated interlocking elements, according to certain aspects.
FIG. 4 is an example of a porous structure having a body that is a perforated tube with integrated interlocking elements, according to certain aspects. In certain aspects, the body of FIG. 4 can be a skin for an internal porous structure.
FIG. 5A is an example of five bodies of units (e.g., porous unit bodies) of a porous structure, according to certain aspects.
FIG. 5B is an example of interlocking elements of a porous structure, according to certain aspects.
FIG. 5C is an example of connecting elements of a porous structure, according to certain aspects.
FIG. 5D is an example of a full porous structure formed from the elements of FIGs. 5A-5C, according to certain aspects.
FIG. 5E is an exploded view of an example of two units of a porous structure, each with a body and interlocking elements, according to certain aspects.
FIG. 6 is an exploded view of a hexagonal porous structure including two units and flexible connecting elements, according to certain aspects. In certain aspects, flexible connecting elements of the porous structure of FIG. 6 can make up a skin.
FIG. 7 is an example of two units of a porous structure connected by flexible connection elements, according to certain aspects.
FIG. 8 is an example of units of a porous structure stacked to form a linear structure and connected by flexible connection elements, according to certain aspects.
FIG. 9 is an example of porous units coupled to form a planar structure connected by flexible connection elements, according to certain aspects.
FIG. 10 is an example of porous units stacked and coupled to form a 3D structure connected by flexible connection elements, according to certain aspects.
FIG. 10A is an example of a spinal spacer formed of porous units stacked and coupled and connected by flexible connection elements, according to certain aspects.
FIG. 11 is an example of a screw with a helicoidal porous structure, according to certain aspects.
FIG. 12 is cut-away view of the example of a screw of FIG. 11. In certain aspects, the screw includes a screw head, screw tip, an outer shell, and a central core.
FIGs. 12A-12C illustrate an example screw including a flexible porous structure and struts configured to sever ingrown bone.
FIG. 13 is an example of a shaft of a screw with a porous structure formed as crossing helixes, according to certain aspects.
FIGs. 14A and 14B are examples of separate units of a porous structure for a shaft of a screw or bolt, according to certain aspects.
FIG. 14C is an example of separate units of FIGs. 14A and 14B in interlocking position, according to certain aspects.
FIG. 14D is an example of a helicoidal connecting element with screw thread for a shaft of a screw or bolt, according to certain aspects.
FIG. 14E is an example of a second helicoidal connecting element for a shaft of a screw or bolt, according to certain aspects.
FIG. 14F is an example of a shaft of a screw or bolt formed from the elements of FIGs. 14C-14E, according to certain aspects.
FIG. 15 is an example of a screw with two parallel helixes as a porous structure, according to certain aspects.
FIG. 16 is an example of a glenoid implant including porous structures, according to certain aspects.
FIG. 17 is an example of a glenoid implant (e.g., for large bone defects) including porous structures, according to certain aspects.
FIG. 18 is an example of a femur implant including porous structures, according to certain aspects.
FIG. 19 is an example of a knee implant including porous structures, according to certain aspects.
FIG. 20 is an example of a dental implant including porous structures, according to certain aspects.
FIG. 21A is an example of a body of an implant for an implant fixation system for bones (e.g, with a small cross section) including porous structures, according to certain aspects.
FIG. 21B is an example of a bolt of an implant for an implant fixation system for bones (e.g, with a small cross section) including porous structures, according to certain aspects.
FIG. 21C is an example of a plug of an implant for an implant fixation system for bones (e.g, with a small cross section) including porous structures, according to certain aspects.
FIG. 21D is an example an implant fixation system for bones (e.g, with a small cross section) including porous structures formed from the elements of FIGs. 21A-21C, according to certain aspects.
FIG. 22 is a schematic view of a prior art medical device.
FIG. 23 is an example of a system for designing and manufacturing 3D objects.
FIG. 24 illustrates a functional block diagram of one example of the computer shown in FIG. 23.
FIG. 25 shows a high-level process for manufacturing a 3D object using an additive manufacturing system.

### DETAILED DESCRIPTION OF INVENTION

The invention is solely defined in the claims. Any subject-matter that is disclosed herein, but which is not defined in the claims, does not form part of the invention. The following description and the accompanying figures are directed to certain specific embodiments. The embodiments described in any particular context are not intended to limit this disclosure to the specified embodiment or to any particular usage. Those of skill in the art will recognize that the disclosed embodiments, aspects, and/or features are not limited to any particular embodiments. For example, reference to "a" layer, component, part, etc., may, in certain aspects, refer to "one or more."

Certain embodiments herein relate to implants that have reduced size as compared to some other porous structures having separate unit bodies, connecting elements, elements that come into contact upon large deformation - e.g., interlocking elements or deformation-restricting elements - and skin.

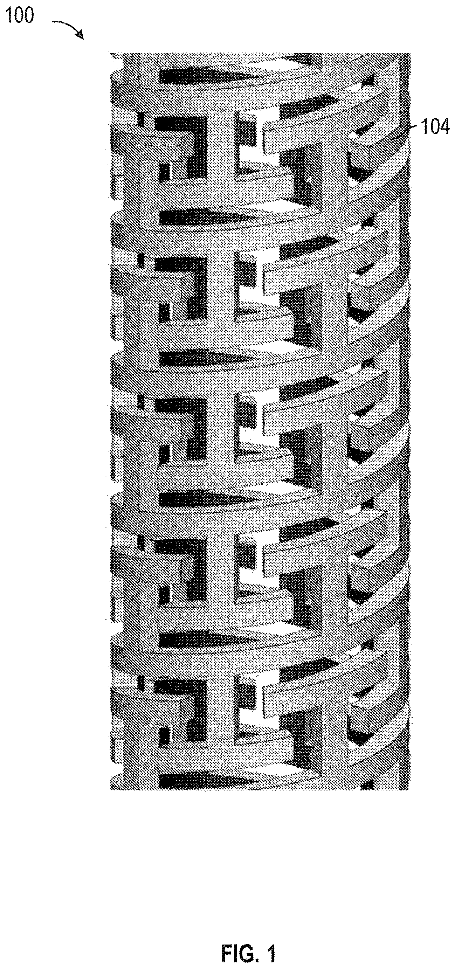

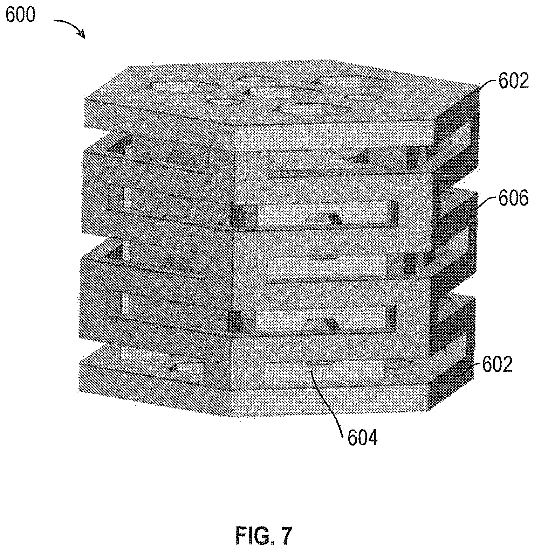

For example, certain embodiments herein provide a porous structure design that further improves on the designs in WO/2017/042366, such as the design in Fig. 13c and described in the specification at p. 20, ll. 15-21 or such as shown in any of FIGs. 1a to 7, 10a to 17b, or 19a-19d and described in the specification at p. 9, 11. 31-p. 15, ll. 18; p. 17, ll. 14-p. 23, ll. 16; or p. 25, ll. 4-28 of WO/2017/042366. In particular, in certain embodiments, instead of structures comprising individual units that are connected by means of connection elements and having interlocking or restricting elements that only make contact upon large deformations, such as in Fig. 13c and described in the specification at p. 20, ll. 15-21 of WO/2017/042366, embodiments of porous structures described herein combine two or more of these elements to achieve a more compact design. One example embodiment includes one or more units that themselves provide the flexible behavior of the connecting elements in WO/2017/042366, such as in Fig. 13c and described in the specification at p. 20, ll. 15-21 of WO/2017/042366, and that internally comprise two or more elements that restrict deformation and contribute to mechanical strength. In other words, the body of the unit at the same time functions as connecting element and optionally even as skin. The restricting elements can be de-coupled elements that come into contact upon a certain level of deformation, such as opposing elements to restrict compression and interlocking elements to restrict extension. A combination of restriction of compression and extension can be obtained by designing interlocking elements that come into contact with each other upon excessive extension and that come into contact with the body of the unit upon excessive compression. Other embodiments are possible.

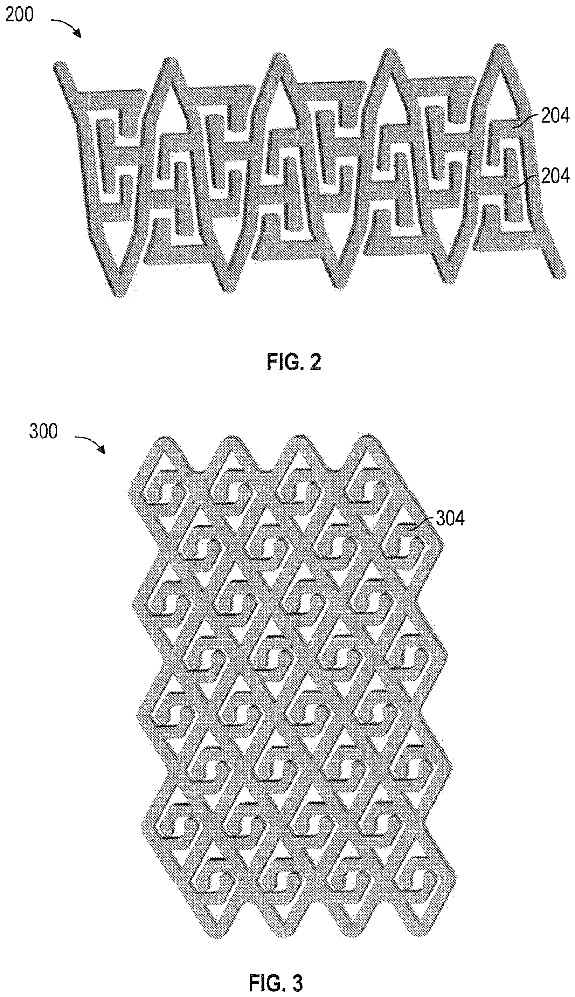

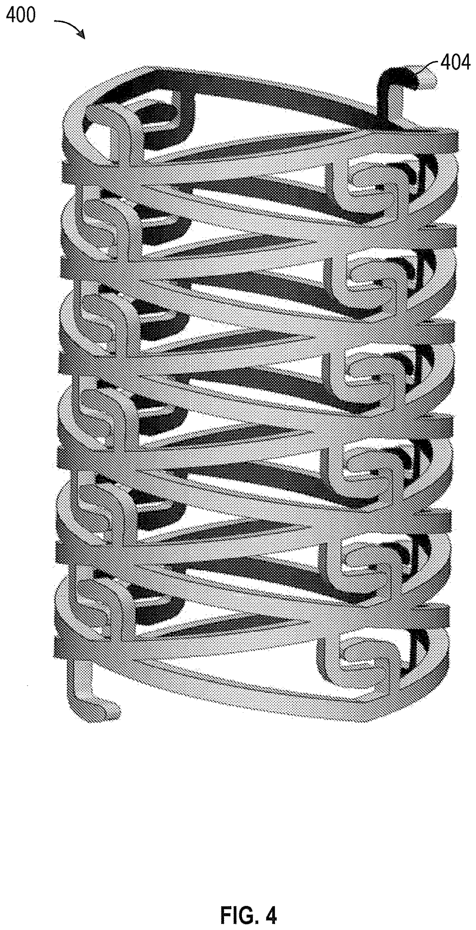

Examples embodiments of such units can have the shape of a helix, such as unit 100 (Figure 1), a zig-zag shape, such as unit 200 (Figure 2), a perforated structure, such as unit 300 (Figure 3, e.g. a honeycomb), a sheet, such as unit 200 or 300 (Figure 2 or Figure 3) or a tube, such as unit 100 or 400 (Figure 1 or Figure 4). Other shapes, such as 3-dimensional structures, layered structures, auxetic structures, prismatic structures, wavy designs, curly designs, staircase designs, stepped designs, corrugated designs, etc., are also possible embodiments. Examples of restricting elements 104, 204, 304, 404 are also shown in Figures 1-4.

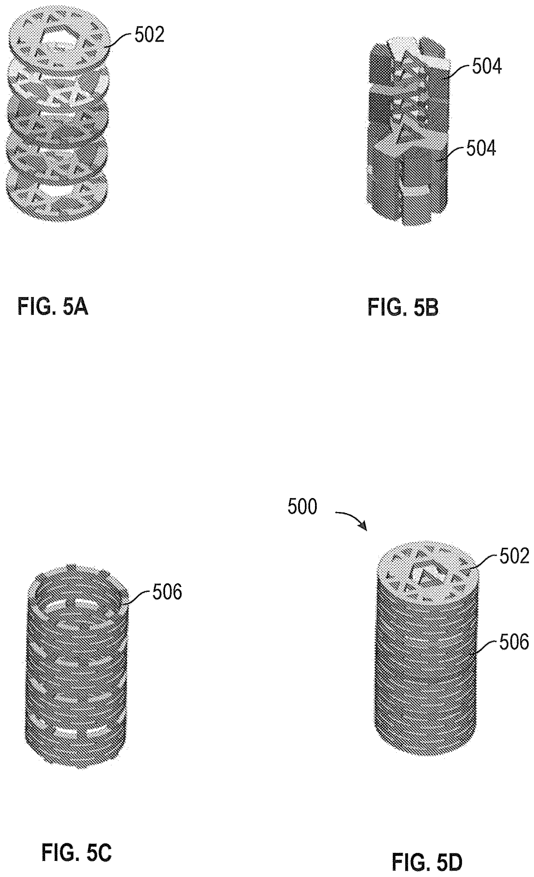

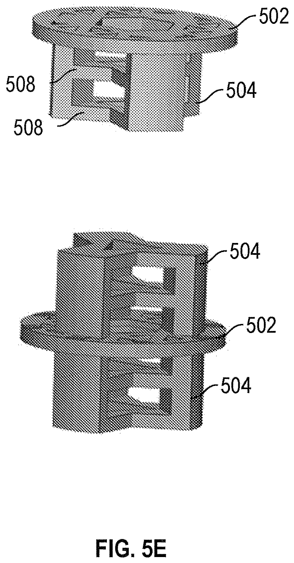

Another example embodiment combines the outer skin with the function of the connecting elements while maintaining identifiable unit bodies. Examples of such embodiments can be seen in Figures 5 to 10.

Figures 5A-E show an example in which the unit bodies 502 take the shape of perforated disks 502 (Figure 5A). Between the disks 502 are interlocking elements 504 (Figure 5B) configured to interlock with one another, and thereby movably couple unit bodies 502. Of each pair of interlocking elements 504, one is attached to one unit body 502 of a pair of adjacent unit bodies 502 and the other to the other unit body 502 of the pair. Figure 5E shows an exploded view of two unit bodies 502 with their interlocking elements 504. In this case the interlocking elements 504 have a layered design with two layers 508, but other designs or designs with a single layer or more than 2 layers are possible. The layers 508 of adjacent interlocking elements 504 may be interposed between one another to movably couple adjacent unit bodies 502. Under normal loads, these interlocking elements 504 of adjacent unit bodies 502 do not come into contact with each other. Upon accidental loads, a larger-than-normal deformation brings the interlocking elements 504 into contact, so that they contribute to the strength of the porous structure 500. The connecting element 506 connecting the unit bodies 502 also acts as a skin 506 (Figure 5C). This skin 506 is designed to provide a flexibility within the desired range. In the example shown in Figure 5C, the desired flexibility is achieved by means of slit-shaped perforations in a staggered configuration. Other designs of connecting element 506, such as spring-like designs, or the designs of Figures 1 and 4 - with or without additional interlocking elements (e.g., interlocking elements 104, 204, 304, 404, etc.) - are possible. Figure 5D shows the assembly of the elements of Figures 5A-C. The example of Figures 5A-E has a circular cross section and a cylindrical shape, but both the cross section and longitudinal shape can be adapted to fit the purpose of the medical device. This example is suited, among others, for the design of bone-defect-filling implants, and in particular for bone-defect-filling implants for elongate bones, such as the radius, ulna, humerus, femur, tibia, fibula, ribs, clavicle, mandible, phalanges, metacarpals, and metatarsals.

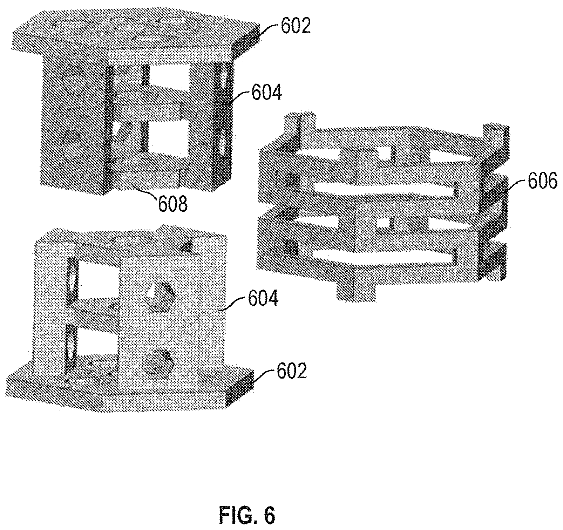

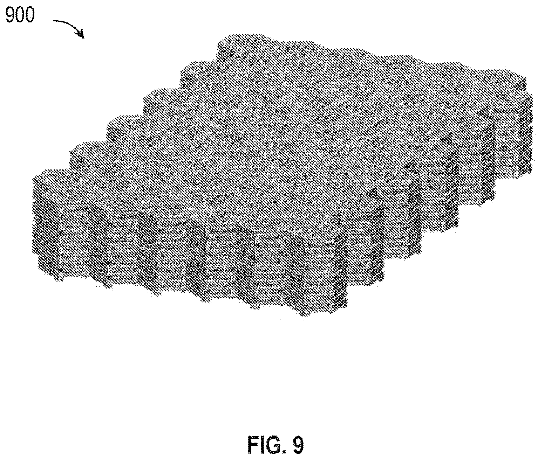

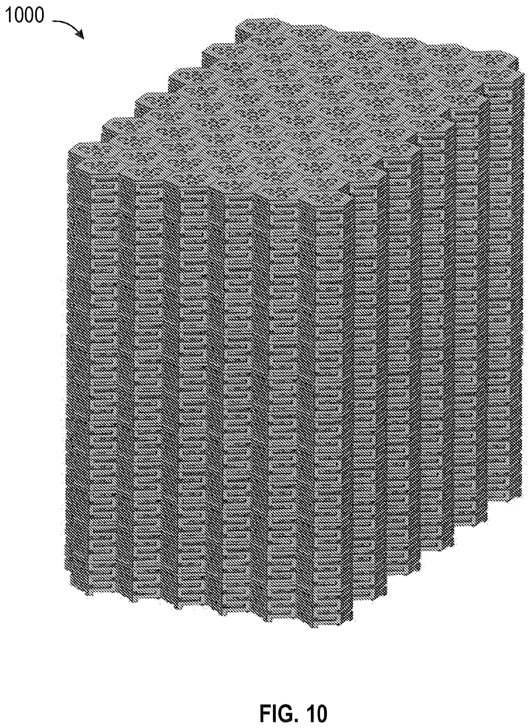

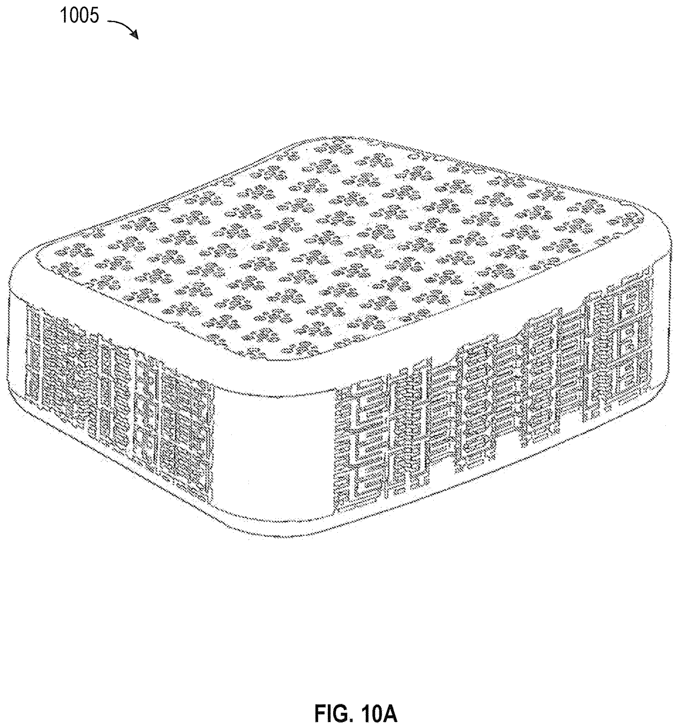

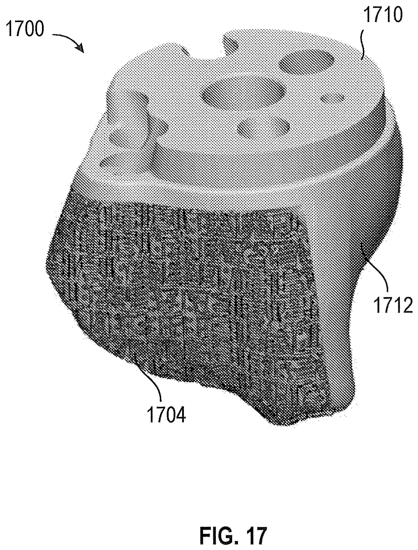

An example of a similar structure 600 with a different cross section is shown in Figures 6-8. For example, the structure 600 includes unit bodies 602, interlocking elements 604, layers 608, and connecting element 606. The different elements of this example adhere to the same principles as the example of Figures 5A-E. However, this example has a polygonal cross section, in particular a hexagonal cross section. Having a more regular-shaped cross section, such as a triangle, rectangle, square, hexagon, etc., allows repeating units of the porous structure to form plate-like structures, such as the plate-like structure 900 that can be seen in Figure 9, or volumes of porous structure, such as the volume of porous structure 1000 that can be seen in Figure 10. Such plate-like structures can be shaped to cover bone-contacting surfaces of implants. Alternatively, such plate-like structures can be shaped to form the body of a plate-like implant, such as a cranial plate. Volumes of porous structure can be shaped or trimmed to form the body or part of the body of an implant, such as the spinal spacer 1005 of Figure 10A, the glenoid implant 1700 of Figure 17 or other implants, such as bone-defect-filling implants.

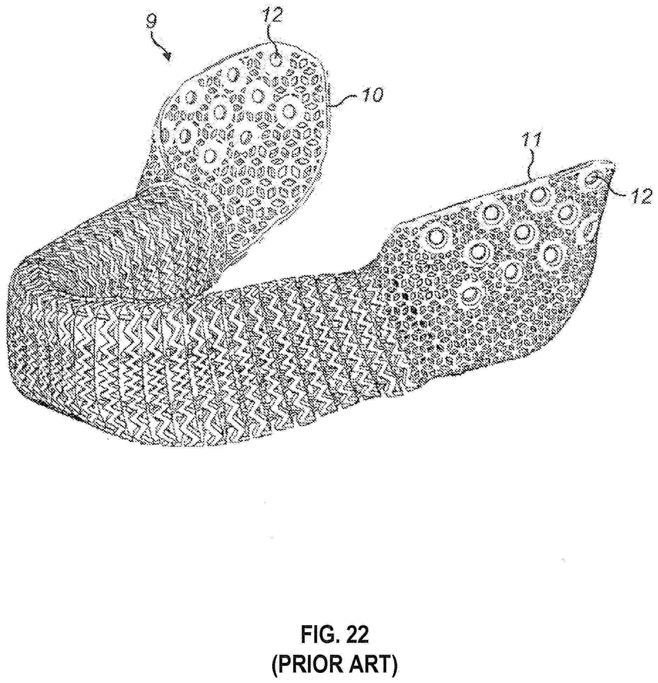

The prior art implant 9 shown in FIG. 22 is connected to the bone by means of end portions 10 and 11. These end portions, though also porous, require so many reinforcements around the many fixation screw holes 12, that they may not achieve the low stiffness under normal loading that is targeted with the rest of the implant 9. This may be problematic, as the bone-implant interface - i.e. the osteotomy plane where the bone has been cut - is not loaded, and all the loads are redirected via a stiffer end portion and stiffer fixation screws towards the side of the bone. In other words, bone ingrowth is less promoted at the bone-implant interface than one would expect.

Certain embodiments herein provide a fixation system that has similar mechanical behavior as the porous structure of WO/2017/042366, such as shown in any of FIGs. 1a to 7, 10a to 17b, or 19a-19d and described in the specification at p. 9, ll. 31-p. 15, 11. 18; p. 17, ll. 14-p. 23, ll. 16; or p. 25, ll. 4-28 of WO/2017/042366: a low stiffness under normal loads and a high strength under higher (i.e. accidental) loads. To accomplish this behavior, in certain embodiments, the fixation system uses an internal structure according to WO/2017/042366, such as shown in any of FIGs. 1a to 7, 10a to 17b, or 19a-19d and described in the specification at p. 9, ll. 31-p. 15,11. 18; p. 17, ll. 14-p. 23, ll. 16; or p. 25, ll. 4-28 of WO/2017/042366, an internal structure according to embodiments described herein, or a combination of both. In certain embodiments, the fixation system comprises one or more stems, pegs, screws and/or bolts, and optionally one or more plugs.

The stems, pegs, screws and/or bolts may have an internal structure according to the porous structure of WO/2017/042366, such as shown in any of FIGs. 1a to 7, 10a to 17b, or 19a-19d and described in the specification at p. 9, ll. 31-p. 15, ll. 18; p. 17, ll. 14-p. 23, ll. 16; or p. 25, ll. 4-28 of WO/2017/042366, or according to the porous structure of embodiments described herein.

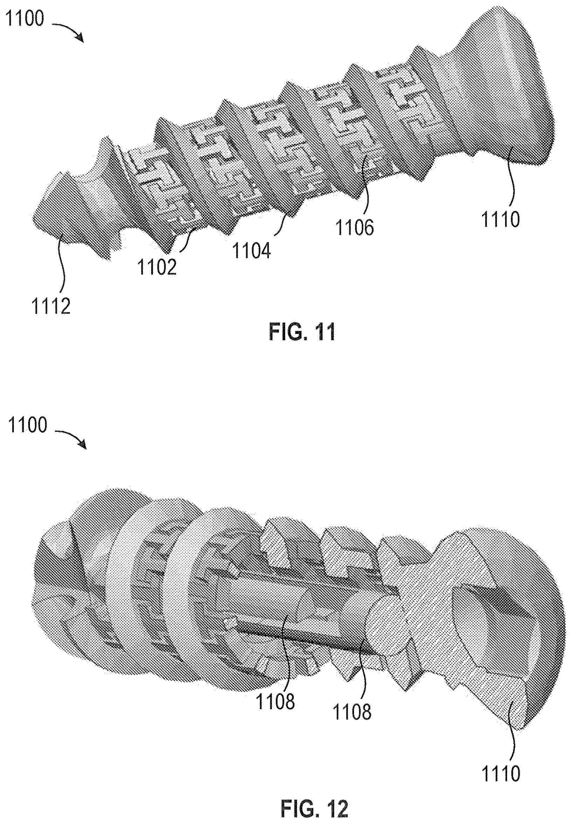

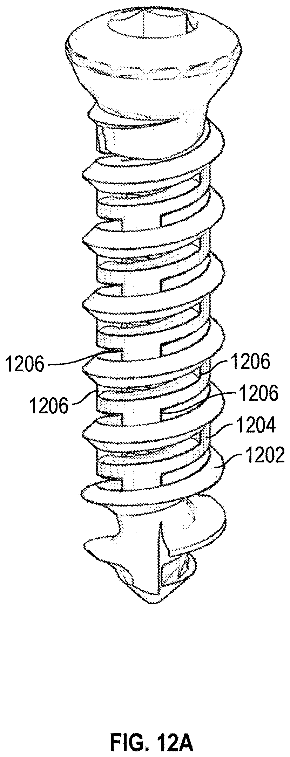

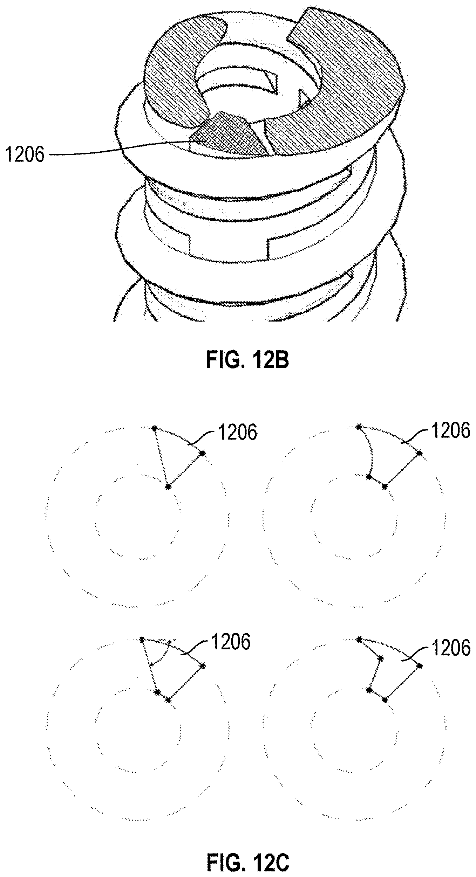

In some embodiments, the shafts of the stems, pegs, screws and/or bolts have an outer shell and an inner core. In certain embodiments, the outer shell can have a structure according to the helicoidal design of Figure 1. In the case of a screw or bolt, a screw thread can then follow the helix. An example of such a screw 1100 is shown in Figure 11-12. As shown, the outer shell comprises a helicoidal body 1102 with screw thread 1104 and interlocking elements 1106. Unfortunately, because the outer shell is a helix, it offers little resistance to torsion until the interlocking elements 1106 touch. However, in some embodiments, torsion stiffness can be increased by having an inner core as shown in Figure 12, comprising longitudinally sliding elements 1108, some connected to the screw head 1110, some to the screw tip 1112, which prevent torsion between head 1110 and tip 1112.

In certain embodiments, the shafts of the pegs, screws and/or bolts have an outer shell with a structure according to the crossing-helix design of Figure 4 (e.g., more than 2 helixes are possible, such as screw 1300 having helixes 1302 and 1304 as shown in Figure 13). A screw thread can then follow one helix. In this embodiment the presence of the crossing helix provides torsional stiffness. The central core can be left open, can be filled with the sliding elements 1108 of Figure 12, or can be filled in another way, e.g. with a porous structure. In certain embodiments, the central core does not prevent the compression and extension of the helicoidal body under normal loads. One example would be a porous structure comprising a multitude of mutually disconnected elements extending inwards from the outer shell.

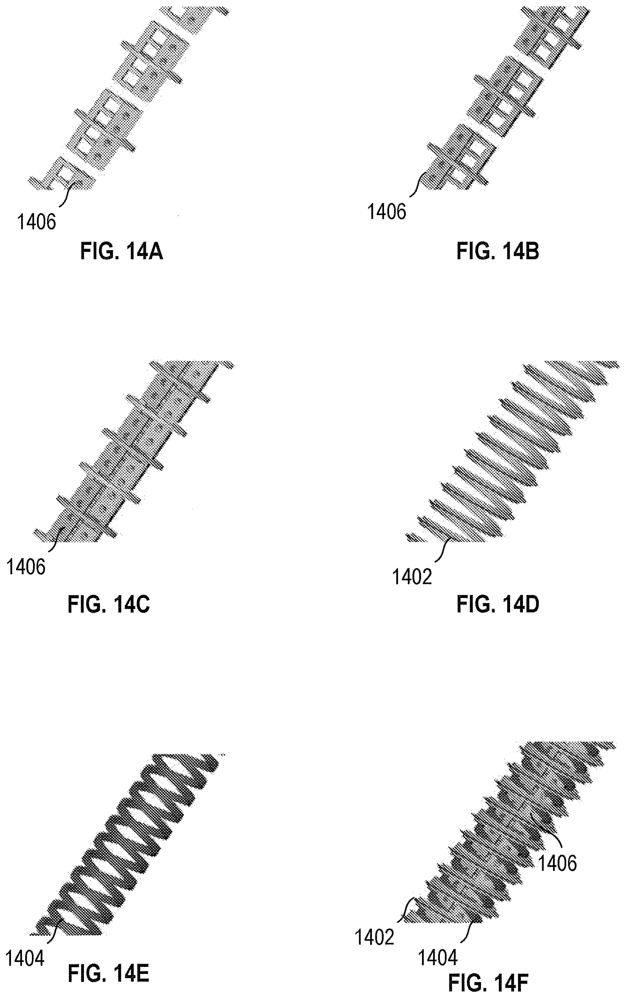

Another embodiment can have a single (e.g., helix body 1402 or 1404 as shown in FIG. 14D or 14E) or crossing-helix body (e.g., helix body 1402 and 1404 as shown in FIG. 14F) occupying the outer shell and interlocking elements 1406 occupying the inner core, as illustrated in Figures 14A-14F.

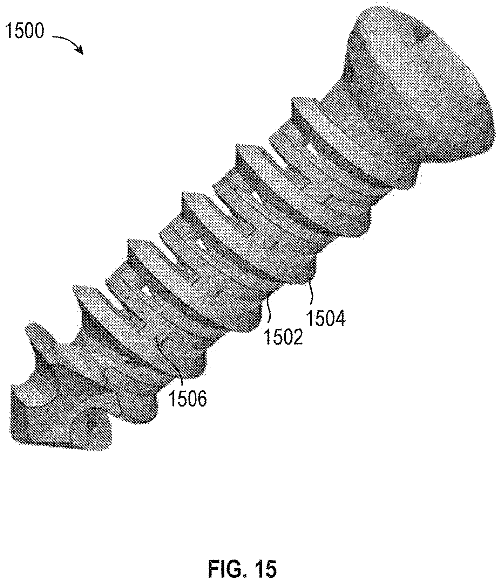

Another embodiment can have two parallel helixes (e.g., helixes 1502 and 1504) connected at intervals 1506 in a staggered configuration as in screw 1500 shown in Figure 15. This design restricts compression upon accidental loads, but not extension.

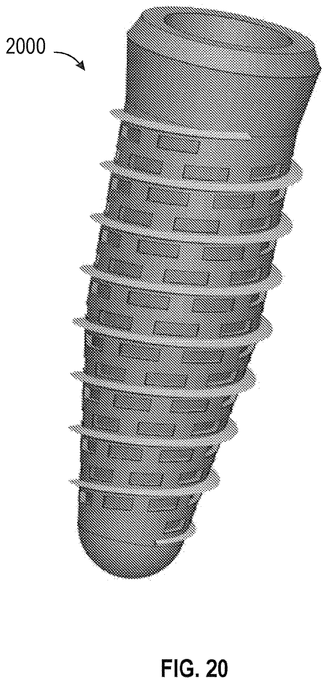

A particular example of a screw according to one embodiment is a dental implant 2000 as can be seen in Figure 20. The shaft of the dental implant can have any of the structures of the previous embodiments. In dental implants, infection due to bacteria present in the mouth is an alleged cause of bone loss. It may therefore be appropriate to provide the implant with an antibacterial coating and/or with a solid, smooth section of shaft where the implant protrudes from the bone.

For certain implants, loads between the implant and the bone are transmitted through shear stresses, which is not natural to bone. Bone is mainly capable of carrying compressive loads. Shear stresses localized around the fixation screws might lead to damage to the bone.

As discussed, in certain embodiments, such as shown in Figures 11-12, a screw includes a flexible porous structure (e.g., the helicoidal design of Figure 1), which promotes bone ingrowth into the screw, which can help in fixation. However, in certain cases, such as revision surgery, it may be desirable to remove/extract the screw from the bone after insertion into the bone. It may further be desirable to reduce damage caused to the bone when removing the screw. Accordingly, certain embodiments herein provide one or more struts along the flexible porous structure configured with sharp edges. The sharp edges are positioned such that as the screw is rotated (e.g., counter-clockwise) so as to extract the screw from the bone, the sharp edges cut or sever the bone ingrown into the screw, thereby allowing the screw to be extracted more cleanly and with less chance of additional damage to the bone. In certain aspects, the ingrown bone that is severed may fall into the screw and therefore be extracted along with the screw.

FIGs. 12A-12C illustrate an example screw including a flexible porous structure and struts configured to sever ingrown bone. As shown, the screw includes a first helicoidal structure 1202 around the screw (e.g., on a mantle of the screw) that comprises a screw thread. The screw further includes a second helicoidal structure 1204 that includes a number of struts 1206. As shown, the struts 1206 are formed along the length of the screw (e.g. correspond to longitudinal struts). The edges of struts 1206 may be formed as sharp edges (e.g., as shown more clearly in FIGs. 12B and 12C) according to any number of appropriate designs. Accordingly, as the screw is rotated and extracted from the bone, the helicoidal structures 1202 and 1204 slide through the bone in the cavity that the screw originally occupied in the bone, and the struts 1206 further collide with bone that has grown into the apertures of the porous structure of the screw. During this extraction, the sharp edge of the struts 1206 severs the bone, allowing the struts 1206 to move through the bone.

In certain aspects, the cross section of the strut, as shown in various embodiments in FIG. 12C, can have an angle of 70 degrees, a lower angle, a higher angle, etc. In certain aspects, some or all struts 1206 and/or additional struts for different screw designs, such as the designs of FIGs. 11-15, include such a sharp edge.

In some embodiments, the fixation system is used to attach an implant to an anatomy part of a patient (e.g. bone). To overcome the weaknesses mentioned, the fixation system may be designed in accordance with the prevailing loads to be expected at the implant/anatomy interface. Bone, for example, models itself to withstand prevailing stresses. An implant, in certain embodiments, may be designed to have an interface with the bone that is substantially perpendicular to the direction of these prevailing stresses so as to effectively transfer the loads from the bone to the implant. The fixation system in certain embodiments is also designed to maintain the transfer of the loads at the interface and not to divert the loads to another location or to convert the loads into other types of loads (e.g. from compression/tension to shear). Pegs, screws, and/or bolts may therefore be applied perpendicularly to the implant/anatomy interface.

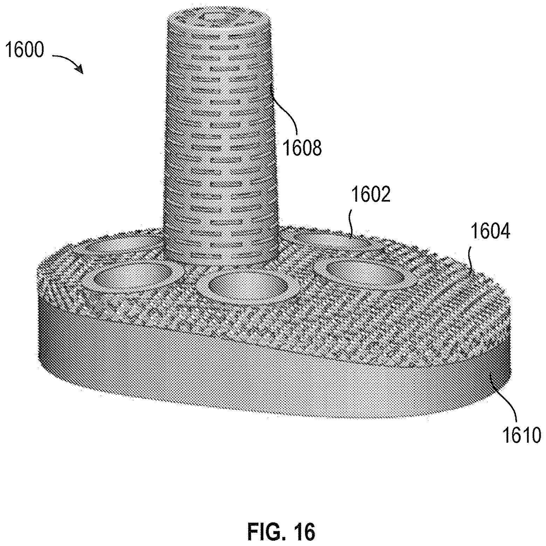

One embodiment of an implant fixation system is a peg or stem attached to the implant, such as can be seen in Figure 16 for a glenoid implant 1600. In one embodiment, the implant 1600 has a solid metal core 1610, perforations 1602 for screw insertion, a porous layer 1604 for bone ingrowth and a porous peg 1608 for stabilization and fixation. To avoid stress shielding and to promote bone ingrowth, one can use one of the porous structures described herein or of WO/2017/042366, such as shown in any of FIGs. 1a to 7, 10a to 17b, or 19a-19d and described in the specification at p. 9, 11. 31-p. 15, 11. 18; p. 17, ll. 14-p. 23, ll. 16; or p. 25, ll. 4-28 of WO/2017/042366, for the peg 1608, the porous layer 1604 (depicted here as a regular porous structure) and/or the fixation screws (not shown in the image).

Another example embodiment of an implant fixation system is the combination of a glenoid implant 1700 such as can be seen in Figure 17 and one or more screws as seen in Figures 11-15. In one embodiment, the glenoid implant 1700 has a solid metal base 1710, a porous structure 1704 according to other aspects of this invention and may be suited for large bone defects. The porous structure 1704 is oriented to accommodate the prevailing loads at the glenoid. At the side 1712, the implant 1700 has a smooth wall to avoid muscles, tendons, nerves and blood vessels to be damaged or irritated as they move over the surface.

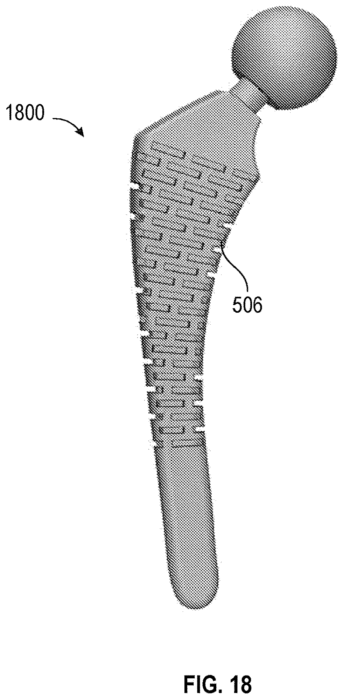

Another example embodiment is a femoral implant 1800 as can be seen in Figure 18. In one embodiment, the structure 506 of Figure 5 is applied to part of the stem of the implant 1800. In this way the stem can be designed to be flexible enough to avoid stress shielding and promote bone ingrowth, while still strong enough to withstand accidental loads.

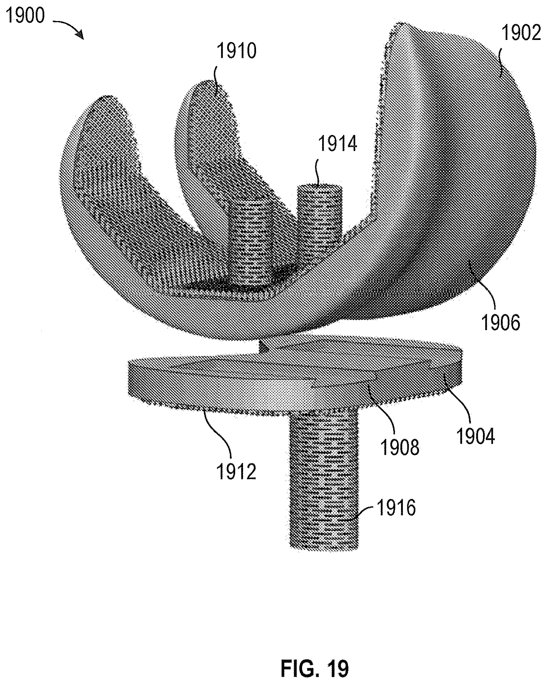

Another example embodiment is a knee implant 1900 as can be seen in Figure 19. In one embodiment, the knee implant 1900, as shown, comprises a metal femoral component 1902, a metal tibial component 1904 and a polymer spacer (not shown). Each metal component has a solid metal core 1906/1908, one or more bone-facing surfaces 1910/1912 and one or more pegs 1914/1916. The pegs 1914/1916 can be cylindrical, conical, fin-like or of any other suitable shape. To promote ingrowth, the bone-facing surfaces 1910/1912 can be covered in a porous structure. The porous structure can be a regular structure (e.g. a repeated unit cell as known in the art), a structure according to WO/2017/042366, such as shown in any of FIGs. 1a to 7, 10a to 17b, or 19a-19d and described in the specification at p. 9, ll. 31-p. 15, 11. 18; p. 17, ll. 14-p. 23, 11. 16; or p. 25, ll. 4-28 of WO/2017/042366, or a structure according to the embodiments discussed herein. The structure may be oriented so as to accommodate the prevailing loading directions. To promote bone ingrowth and prevent stress shielding, the pegs may be designed to comprise one of the porous structures of WO/2017/042366, such as shown in any of FIGs. 1a to 7, 10a to 17b, or 19a-19d and described in the specification at p. 9, ll. 31-p. 15, 11. 18; p. 17, ll. 14-p. 23, ll. 16; or p. 25, ll. 4-28 of WO/2017/042366, or of the embodiments discussed herein.

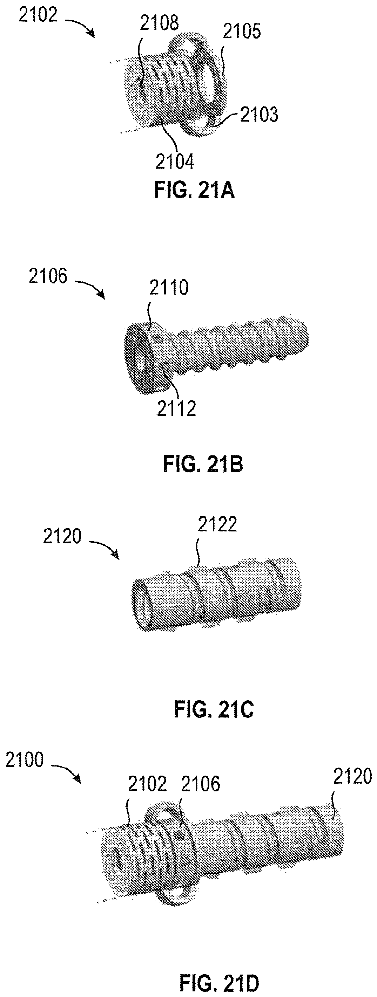

One embodiment, such as shown in FIG. 21D, provides a fixation system 2100 for elongate bones, such as a mandible or the diaphysis of a femur, tibia, fibula, humerus, radius, ulna, rib, clavicle, metacarpal, metatarsal, phalange. Particularly in bones with a smaller cross section it can be a challenge to fixate a bone-replacing implant with a fixation system with an interface perpendicular to the axis of the bone and fixation screws substantially parallel to the axis of the bone. According to this embodiment, the implant fixation system 2100 comprises the following elements:
- a body 2102 as shown in FIG. 21A. This body 2102 replaces a section of the bone. In an embodiment, the body 2102 has a porous structure according to WO/2017/042366, such as shown in any of FIGs. 1a to 7, 10a to 17b, or 19a-19d and described in the specification at p. 9, ll. 31-p. 15, ll. 18; p. 17, ll. 14-p. 23, ll. 16; or p. 25, ll. 4-28 of WO/2017/042366, or to embodiments disclosed in the present application. The body 2102 has an interface 2104 with the bone that is substantially perpendicular to the axis of the bone. The body 2102 includes connecting elements 2103 that connect interface 2104 to a bolt or screw receiving element 2105. The space between interface 2104 and receiving element 2105 form a volume for a bolt or screw to lie within;
- a bolt or screw 2106 as shown in FIG. 21B. To overcome the challenge of the limited dimensions and still be able to align the bolt or screw 2106 within the bone/implant interface and substantially parallel to the axis of the bone, the head 2110 of the bolt or screw 2106 may lie within the volume of the body 2102. The interface 2104 and receiving element 2105 restrict movement in the direction perpendicular to the axis of the bone of the bolt or screw 2106. The body 2102 may have an aperture 2108 extending further along the axis of the bolt or screw 2106 to allow the insertion of a screw driver. Alternatively, the head 2110 of the bolt or screw 2106 can be made accessible from the side, so that the bolt or screw 2106 can be fastened with a wrench or with a pin that grabs into a series of apertures 2112 in the head 2110 of the bolt or screw 2106. In one embodiment, the bolt or screw 2106 has a porous structure as shown for the stems, pegs and screws above;
- optionally a plug 2120 as shown in FIG. 21C. Particularly when there is no space to provide an aperture 2108 for a screw driver and the bolt or screw 2106 has to be fastened from the side, it's difficult for the surgeon to put sufficient pressure on the bolt or screw 2106 to drive it into the bone. In one embodiment, the implant fixation system therefore also comprises a plug 2120. The hole for the plug 2120 can be pre-drilled, such as using a (patient-specific) drill guide. The plug 2120 can then be inserted into the hole, for example manually or using a hammer. The plug 2120 comprises external features 2122 to limit rotation (e.g. fins parallel to the axis of the plug). The plug 2120 also comprises at least one element to prevent extraction when the implant is fixed to the bone. This can be one or more elements that are pushed outward when the bolt 2106 is driven into the plug 2120. Alternatively, it can be one or more anchoring screws or pins inserted at roughly 90° from the axis of the plug 2120, such as are known from intramedullary nails. In one embodiment, the plug 2120 has a porous structure as shown for the stems, pegs and/or screws above.

One advantage of using a bolt 2106 and plug 2120 is that the plug 2120 can be designed to fit the shape of the bone. For example, the plug 2120 can be given a diameter best suitable for the cross section of the bone. For example, the plug 2120 can be dimensioned to fit between the cortical walls of the bone.

An important advantage of using an implant fixation system of which (e.g. all) elements have porous structures as described herein, is that not only the design of the implant, but also the fixation system promotes bone ingrowth.

Certain embodiments provide a flexible porous structure for implantation into bone. In certain embodiments, the structure includes a helicoidal structure, such as shown in any one of FIGs. 1, 4, 5C, 5D, 6-13, 14D-16, 19, or 20. Further, the structure includes a plurality of interlocking elements coupled to the helicoidal structure, the helicoidal structure being configured to connect the plurality of interlocking elements, such as shown in any one of FIGs. 1, 4, 5A, 5B, 5D-16, 19, or 20. Each of the plurality of interlocking elements is configured to have space between the interlocking element and the helicoidal structure and space between the interlocking element and adjacent interlocking elements when in a neutral position. The plurality of interlocking elements are configured to contact the helicoidal structure when a compressive force is applied to the flexible porous structure thereby restricting compression of the helicoidal structure. The plurality of interlocking elements are configured to contact adjacent interlocking elements when an extension force is applied to the flexible porous structure thereby restricting extension of the helicoidal structure.

In certain embodiments, the helicoidal structure and the plurality of interlocking elements form a surface of a hollow cylinder, such as shown in FIG. 1, 4, 11-13, or 15. In certain embodiments each of the plurality of interlocking elements comprises a T structure, and adjacent interlocking elements are oriented in a mirrored fashion from one another, such as shown in FIG. 1, 11, or 12.

In certain embodiments, the helicoidal structure forms a surface of a cylinder, and the plurality of interlocking elements are positioned in an interior of the cylinder, such as shown in FIGs. 5A-10 or 14F.

In certain embodiments, the flexible porous structure comprises a screw, wherein the helicoidal structure comprises an outer shell of the screw, such as shown in FIGs. 11-15 or 20. In certain embodiments, the screw includes a screw thread formed on the outer shell, such as shown in FIGs. 11-15 or 20. In certain embodiments, the screw thread aligns with the helicoidal structure, such as shown in FIGs. 11-12C, 15, or 20. In certain embodiments, the screw thread forms a crossing helix design with the helicoidal structure, such as shown in FIG. 13.

In certain embodiments, the screw comprises an inner core comprising a plurality of sliding elements including a first sliding element coupled to a head of the screw and a second sliding element coupled to a tip of the screw, wherein: the plurality of sliding elements is configured to have space between the first sliding element and the second sliding element when in the neutral position, and the first sliding element is configured to contact the second sliding element when a force (e.g., the compressive force, a torsional force, etc.) is applied to the flexible porous structure thereby restricting compression and/or torsion of the helicoidal structure, such as shown in FIG 12. In certain such aspects, the first and second sliding elements are configured to resist torsion of the screw around a longitudinal axis of the screw.

In certain embodiments, the screw comprises an inner core comprising a plurality of sliding elements including a first sliding element coupled to a head of the screw and a second sliding element coupled to a tip of the screw, wherein: the first and second sliding elements are configured to slide with respect to one another along a longitudinal axis of the screw, such as shown in FIG 12. In certain such aspects, the first and second sliding elements are configured to resist torsion of the screw around the longitudinal axis.

In certain embodiments, the helicoidal structure comprises at least one strut (e.g., formed along a length of the screw), the at least one strut comprising a sharp edge, such as shown in FIG. 12A-12C.

Certain embodiments provide an implant fixation system including a body comprising a porous structure, the body configured to interface with a bone perpendicular to an axis of the bone, such as shown in FIG. 21A. The implant fixation system further includes a screw comprising a head, such as shown in FIG 21B. The head is configured to lie within a volume of the body while a portion of the screw extends away from the body, such as shown in FIG. 21D. The body is configured to restrict movement of the head within the body along the axis of the bone.

In certain embodiments, the implant fixation system further includes a plug configured to receive the portion of the screw, wherein the plug is configured to be inserted in a hole formed into bone, such as shown in FIG. 21C. In certain embodiments, the plug comprises external features configured to limit rotation of the plug in the hole, such as shown in FIG. 21C. In certain embodiments, the head comprises a second porous structure, such as shown in FIG. 21D.

In certain aspects, embodiments described herein, such as of implants, porous structures, flexible porous implant fixation systems, etc., may be manufactured using an additive manufacturing AM process.

AM processes are a material-addition approach to building parts, typically starting from a base material in liquid, solid sheet or powder form and consolidating the added material locally, in layer-by-layer fashion. Since the emergence of the first AM processes in the early 1990's, AM processes have been used as an alternative to conventional material-removal techniques such as milling, cutting or drilling or molding techniques, such as injection molding or extrusion molding, and have been shown to be especially effective in producing complex parts in a relatively short time, without dedicated tools such as molds or dies.

Among the best-known AM techniques are stereolithography (SLA), 3D-printing (3D-P), Selective Laser Sintering (SLS), Selective Heat Sintering (SHS), Selective Laser Melting (SLM), Direct Metal Laser Sintering (DMLS), Laser Beam Melting (LBM), and Electron Beam Melting (EBM). The techniques vary according to the tools used for consolidating the layers of a part, and according to materials that can be used in the techniques.

The systems and methods described herein may be performed using various additive manufacturing and/or three-dimensional (3D) printing systems and techniques. Typically, additive manufacturing techniques start from a digital representation (e.g., CAD file, such as STL, DWG, DXF, etc., mesh based model, voxel based model, etc.) of the 3D object to be formed. Generally, the digital representation is divided into a series of cross-sectional layers (e.g., perpendicularly to the Z-direction, meaning parallel to a build platform), or "slices," which are overlaid to form the object as a whole. The layers represent the 3D object, and may be generated using additive manufacturing modeling software executed by a computing device. For example, the software may include computer aided design and manufacturing (CAD/CAM) software. Information about the cross-sectional layers of the 3D object may be stored as cross-sectional data. An additive manufacturing (e.g., 3D printing) machine or system utilizes the cross-sectional data for the purpose of building the 3D object on a layer by layer basis. Accordingly, additive manufacturing allows for fabrication of 3D objects directly from computer generated data of the objects, such as computer aided design (CAD) files or STL files. Additive manufacturing provides the ability to quickly manufacture both simple and complex parts without tooling and without the need for assembly of different parts.

Additive manufacturing processes generally include providing energy from an energy source (e.g., a laser, an electron beam, etc.) to solidify (e.g., polymerize) layers of building material (e.g., plastic, metal, etc.). For example, the additive manufacturing machine may selectively apply energy from an energy source to (e.g., scan) the building material based on a job file. The job file may include information regarding slices of a digital representation of an object or objects to be built using an additive manufacturing process. For example, 3D objects represented by CAD files may be arranged in a virtual build volume corresponding to the build volume of an additive manufacturing device. Optionally, support structures may be added to the 3D objects in the virtual build volume (e.g., to improve build quality, heat dissipation, reduce deformation, etc.) The resulting 3D objects may be divided into layers or slices, as discussed. The job file, accordingly, may include slices (e.g., a stack of slices) of the 3D objects, and parameters of the additive manufacturing machine for building the 3D objects.

For example, for each slice, the job file may include information regarding a scanning pattern for the energy source to apply energy to (e.g., laser to scan, electron beam to scan, etc.) the physical layer of building material corresponding to that slice. It should be noted that as discussed herein, the terms slice and layer may be used interchangeably. The scanning pattern may include one or more vectors that each indicates a spatial position to apply the energy to the layer of building material and a direction to apply the energy to the building material (e.g., a direction to move the laser beam, electron beam, or other energy source over the building material while scanning).

An additive manufacturing machine builds an object on a layer by layer basis by applying energy to (e.g., scanning) the layers of building material according to the scanning pattern for each individual layer as indicated in a job file. For example, the additive manufacturing machine may scan a first layer of physical building material corresponding to a first slice of a digital representation of an object according to the scanning pattern for the first slice. The additive manufacturing machine may then scan a second layer of building material corresponding to a second slice adjacent to the first slice according to the scanning pattern for the second slice. The additive manufacturing machine continues scanning layers of building material corresponding to all the slices in the job file, until the layer corresponding to the last slice is scanned.

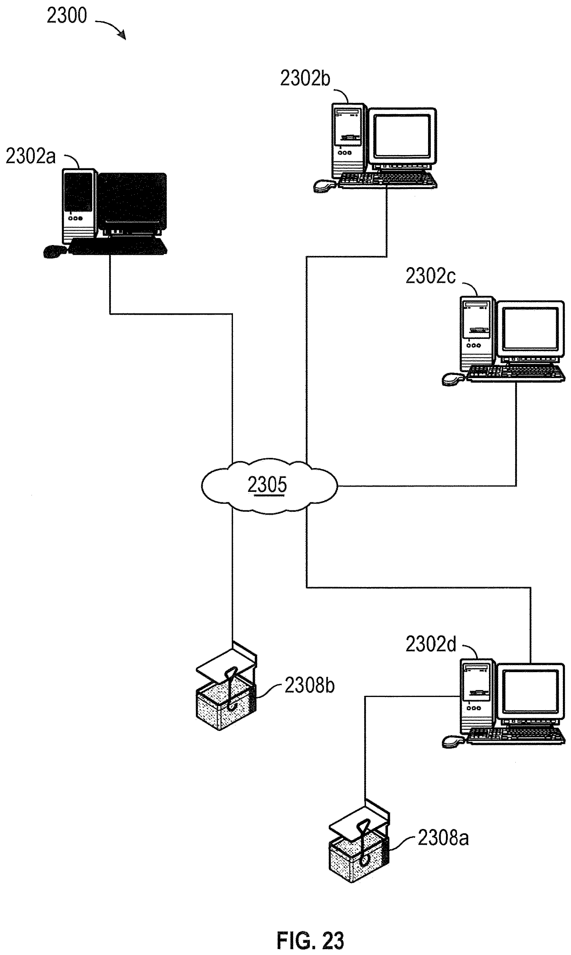

Embodiments of the invention may be practiced within a system for designing and manufacturing 3D objects. Turning to Figure 23, an example of a computer environment suitable for the implementation of 3D object design and manufacturing is shown. The environment includes a system 2300. The system 2300 includes one or more computers 2302a-2302d, which can be, for example, any workstation, server, or other computing device capable of processing information. In some aspects, each of the computers 2302a-2302d can be connected, by any suitable communications technology (e.g., an internet protocol), to a network 2305 (e.g., the Internet). Accordingly, the computers 2302a-2302d may transmit and receive information (e.g., software, digital representations of 3-D objects, commands or instructions to operate an additive manufacturing device, etc.) between each other via the network 2305.

The system 2300 further includes one or more additive manufacturing devices (e.g., 3-D printers) 23025a-23025b. As shown the additive manufacturing device 23025a is directly connected to a computer 2302d (and through computer 2302d connected to computers 2302a-2302c via the network 2305) and additive manufacturing device 23025b is connected to the computers 2302a-2302d via the network 2305. Accordingly, one of skill in the art will understand that an additive manufacturing device 23025 may be directly connected to a computer 2302, connected to a computer 2302 via a network 2305, and/or connected to a computer 2302 via another computer 2302 and the network 2305.

It should be noted that though the system 2300 is described with respect to a network and one or more computers, the techniques described herein also apply to a single computer 2302, which may be directly connected to an additive manufacturing device 23025. Any of the computers 2302a-2302d may be configured to function as the computing device described with respect to FIGs. 1-21. Further, any of the computers 2302a-2302d may be configured to perform the operations described herein.

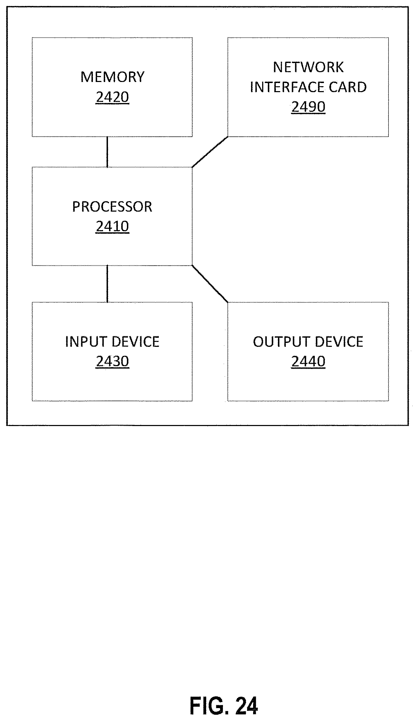

FIG. 24 illustrates a functional block diagram of one example of a computer of FIG. 23. The computer 2302a includes a processor 2410 in data communication with a memory 2420, an input device 2430, and an output device 2440. In some embodiments, the processor is further in data communication with an optional network interface card 2490. Although described separately, it is to be appreciated that functional blocks described with respect to the computer 2302a need not be separate structural elements. For example, the processor 2410 and memory 2420 may be embodied in a single chip.

The processor 2410 can be a general purpose processor, a digital signal processor (DSP), an application specific integrated circuit (ASIC), a field programmable gate array (FPGA) or other programmable logic device, discrete gate or transistor logic, discrete hardware components, or any suitable combination thereof designed to perform the functions described herein. A processor may also be implemented as a combination of computing devices, e.g., a combination of a DSP and a microprocessor, a plurality of microprocessors, one or more microprocessors in conjunction with a DSP core, or any other such configuration.

The processor 2410 can be coupled, via one or more buses, to read information from or write information to memory 2420. The processor may additionally, or in the alternative, contain memory, such as processor registers. The memory 2420 can include processor cache, including a multi-level hierarchical cache in which different levels have different capacities and access speeds. The memory 2420 can also include random access memory (RAM), other volatile storage devices, or non-volatile storage devices. The storage can include hard drives, optical discs, such as compact discs (CDs) or digital video discs (DVDs), flash memory, floppy discs, magnetic tape, and Zip drives.

The processor 2410 also may be coupled to an input device 2430 and an output device 2440 for, respectively, receiving input from and providing output to a user of the computer 2302a. Suitable input devices include, but are not limited to, a keyboard, buttons, keys, switches, a pointing device, a mouse, a joystick, a remote control, an infrared detector, a bar code reader, a scanner, a video camera (possibly coupled with video processing software to, e.g., detect hand gestures or facial gestures), a motion detector, or a microphone (possibly coupled to audio processing software to, e.g., detect voice commands). Suitable output devices include, but are not limited to, visual output devices, including displays and printers, audio output devices, including speakers, headphones, earphones, and alarms, additive manufacturing devices, and haptic output devices.

The processor 2410 further may be coupled to a network interface card 2490. The network interface card 2490 prepares data generated by the processor 2410 for transmission via a network according to one or more data transmission protocols. The network interface card 2490 also decodes data received via a network according to one or more data transmission protocols. The network interface card 2490 can include a transmitter, receiver, or both. In other embodiments, the transmitter and receiver can be two separate components. The network interface card 2490, can be embodied as a general purpose processor, a digital signal processor (DSP), an application specific integrated circuit (ASIC), a field programmable gate array (FPGA) or other programmable logic device, discrete gate or transistor logic, discrete hardware components, or any suitable combination thereof designed to perform the functions described herein.

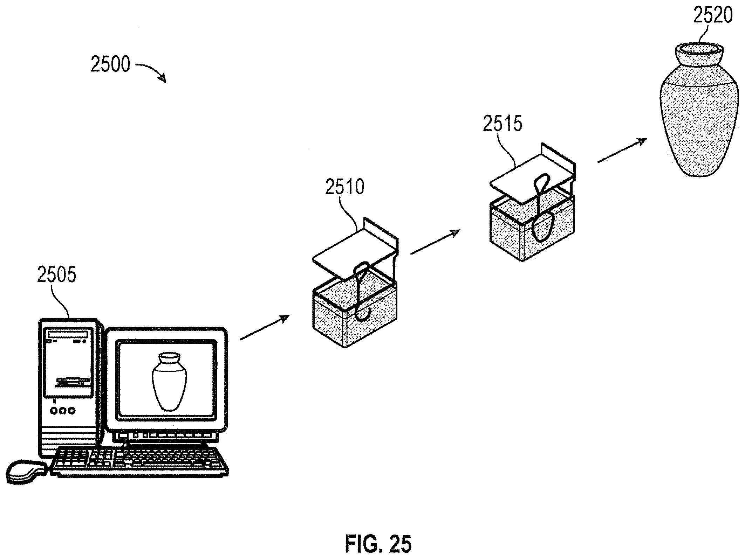

FIG. 25 illustrates a process 2500 for manufacturing a 3-D object or device. As shown, at a step 2505, a digital representation of the object is designed using a computer, such as the computer 2302a. For example, 2-D or 3-D data may be input to the computer 2302a for aiding in designing the digital representation of the 3-D object. Continuing at a step 2510, information is sent from the computer 2302a to an additive manufacturing device, such as additive manufacturing device 23025, and the device 23025 commences the manufacturing process in accordance with the received information. At a step 2515, the additive manufacturing device 25025 continues manufacturing the 3-D object using suitable materials, such as a liquid resin. At a step 2520, the object is finally built.

These suitable materials may include, but are not limited to a photopolymer resin, polyurethane, methyl methacrylate-acrylonitrile-butadiene-styrene copolymer, resorbable materials such as polymer-ceramic composites, metals, metal alloys, etc. Examples of commercially available materials are: DSM Somos^{®} series of materials 7100, 8100, 9100, 9420, 10100, 11100, 12110, 14120 and 15100 from DSM Somos; ABSplus-P430, ABSi, ABS-ESD7, ABS-M30, ABS-M30i, PC-ABS, PC ISO, PC, ULTEM 9085, PPSF and PPSU materials from Stratasys; Accura Plastic, DuraForm, CastForm, Laserform and VisiJet line of materials from 3D-Systems; the PA line of materials, PrimeCast and PrimePart materials and Alumide and CarbonMide from EOS GmbH, Aluminum, CobaltChrome and Stainless Steel materials, MarangingSteel, Nickel Alloy, Titanium, and Titanium alloys. The VisiJet line of materials from 3-Systems may include Visijet Flex, Visijet Tough, Visijet Clear, Visijet HiTemp, Visijet e-stone, Visijet Black, Visijet Jewel, Visijet FTI, etc. Examples of other materials may include Objet materials, such as Objet Fullcure, Objet Veroclear, Objet Digital Materials, Objet Duruswhite, Objet Tangoblack, Objet Tangoplus, Objet Tangoblackplus, etc. Another example of materials may include materials from the Renshape 5000 and 7800 series. Further, at a step 2520, the 3-D object is generated.

Various embodiments disclosed herein provide for the use of computer software being executed on a computing device. A skilled artisan will readily appreciate that these embodiments may be implemented using numerous different types of computing devices, including both general-purpose and/or special-purpose computing system environments or configurations. Examples of well-known computing systems, environments, and/or configurations that may be suitable for use in connection with the embodiments set forth above may include, but are not limited to, personal computers, server computers, hand-held or laptop devices, multiprocessor systems, microprocessor-based systems, programmable consumer electronics, network PCs, minicomputers, mainframe computers, distributed computing environments that include any of the above systems or devices, and the like. These devices may include stored instructions, which, when executed by a microprocessor in the computing device, cause the computer device to perform specified actions to carry out the instructions. As used herein, instructions refer to computer-implemented steps for processing information in the system. Instructions can be implemented in software, firmware or hardware and include any type of programmed step undertaken by components of the system.

A microprocessor may be any conventional general purpose single- or multi-chip microprocessor such as a Pentium^{®} processor, a Pentium^{®} Pro processor, a 8051 processor, a MIPS^{®} processor, a Power PC^{®} processor, or an Alpha^{®} processor. In addition, the microprocessor may be any conventional special purpose microprocessor such as a digital signal processor or a graphics processor. The microprocessor typically has conventional address lines, conventional data lines, and one or more conventional control lines.

Aspects and embodiments of the inventions disclosed herein may be implemented as a method, apparatus or article of manufacture using standard programming or engineering techniques to produce software, firmware, hardware, or any combination thereof. The term "article of manufacture" as used herein refers to code or logic implemented in hardware or non-transitory computer readable media such as optical storage devices, and volatile or non-volatile memory devices or transitory computer readable media such as signals, carrier waves, etc. Such hardware may include, but is not limited to, field programmable gate arrays (FPGAs), application-specific integrated circuits (ASICs), complex programmable logic devices (CPLDs), programmable logic arrays (PLAs), microprocessors, or other similar processing devices.

Various embodiments disclosed herein may be implemented using a computer or computer control system. A skilled artisan will readily appreciate that these embodiments may be implemented using numerous different types of computing devices, including both general-purpose and special-purpose computing-system environments or configurations. Examples of well-known computing systems, environments, and/or configurations that may be suitable for use in connection with the embodiments set forth above may include, but are not limited to, personal computers, server computers, hand-held or laptop devices, multiprocessor systems, microprocessor-based systems, programmable consumer electronics, network PCs, minicomputers, mainframe computers, distributed computing environments that include any of the above systems or devices, and the like. These devices may include stored instructions, which, when executed by a microprocessor in the computing device, cause the computer device to perform specified actions to carry out the instructions. As used herein, instructions refer to computer-implemented steps for processing information in the system. Instructions can be implemented in software, firmware or hardware and include any type of programmed step undertaken by components of the system.

A microprocessor may be any conventional general purpose single- or multi-chip microprocessor such as a Pentium^{®} processor, a Pentium^{®} Pro processor, a 8051 processor, a MIPS^{®} processor, a Power PC^{®} processor, or an Alpha^{®} processor. In addition, the microprocessor may be any conventional special purpose microprocessor such as a digital signal processor or a graphics processor. The microprocessor typically has conventional address lines, conventional data lines, and one or more conventional control lines.

Aspects and embodiments of the inventions disclosed herein may be implemented as a method, apparatus or article of manufacture using standard programming or engineering techniques to produce software, firmware, hardware, or any combination thereof. The term "article of manufacture" as used herein refers to code or logic implemented in hardware or nontransitory computer readable media such as optical storage devices, and volatile or non-volatile memory devices or transitory computer readable media such as signals, carrier waves, etc. Such hardware may include, but is not limited to, field programmable gate arrays (FPGAs), application-specific integrated circuits (ASICs), complex programmable logic devices (CPLDs), programmable logic arrays (PLAs), microprocessors, or other similar processing devices.

## Claims

1. A flexible porous structure (500, 600) for implantation into bone, the structure comprising:
a plurality of unit bodies (502, 602);
an outer skin (506, 606); and
a plurality of interlocking elements (504, 604),
wherein:
the plurality of unit bodies comprises at least one pair of adjacent unit bodies, said pair of adjacent unit bodies comprising a first unit body and a second unit body;
the plurality of interlocking elements comprises at least one pair of adjacent interlocking elements, said pair of adj acent interlocking elements comprising a first interlocking element attached to the first unit body, and a second interlocking element attached to the second unit body;
the first and second interlocking elements are configured to not come into contact with each other in a neutral position; and
the first and second interlocking elements are configured to contact each other under a load,
**characterized in that** the outer skin (506, 606) is configured to flexibly connect the plurality of unit bodies.

2. The flexible porous structure of claim 1, wherein the unit bodies are perforated disks.

3. The flexible porous structure of claim 1 or 2, wherein each of the plurality of interlocking elements comprises a layered structure, and wherein layers of adjacent interlocking elements are interposed between one another.

4. The flexible porous structure of any one of claims 1 to 3, wherein the outer skin comprises a spring-like design.

5. The flexible porous structure of any one of claims 1 to 3, wherein the outer skin comprises a helix design or a crossing-helix design.

6. The flexible porous structure of claim 4 or 5, wherein the outer skin further comprises additional interlocking elements.

7. The flexible porous structure of any one of claims 1 to 3, wherein the outer skin comprises slit-shaped perforations in a staggered configuration.

8. The flexible porous structure of any of claims 1 to 7, wherein the unit bodies are stacked in one direction.

9. The flexible porous structure of any one of claims 1 to 8, wherein the flexible porous structure comprises a cylindrical shape.

10. The flexible porous structure of any one of claims 1 to 8, wherein the flexible porous structure comprises a polygonal cross section.

11. The flexible porous structure of claim 10, wherein the polygonal cross section comprises a triangle, square, rectangle or hexagon.

12. A flexible porous structure (900) for implantation into bone, comprising a platelike structure, said plate-like structure comprising a plurality of flexible porous structures according to claim 10 or 11.

13. A flexible porous structure (1000) for implantation into bone, comprising a volume, said volume comprising a plurality of flexible porous structures according to claim 10 or 11.

14. An implant,
wherein the implant is a bone-defect-filling implant for an elongate bone, such as a radius, an ulna, a humerus, a femur, a tibia, a fibula, a rib, a clavicle, a mandible, a phalange, a metacarpal or a metatarsal, and said bone-defect-filling implant comprises a flexible porous structure according to any one of claims 1-9;
wherein the implant comprises a flexible porous structure according to claim 12, wherein the plate-like structure covers a bone-contacting surface of the implant;
wherein the implant comprises a flexible porous structure according to claim 12, wherein the implant is a plate-like implant, and wherein the plate-like structure forms a body of the implant; or
wherein the implant comprises a flexible porous structure according to claim 13, wherein the volume forms at least part of a body of the implant.

## Patentansprüche

1. Flexible poröse Struktur (500, 600) zur Einpflanzung in einen Knochen, wobei die Struktur aufweist:
eine Mehrzahl von Einheitskörpern (502, 602),
eine Außenhaut (506, 606), und
eine Mehrzahl von Verriegelungselementen (504, 604),
wobei:
die Mehrzahl von Einheitskörpern mindestens ein Paar benachbarter Einheitskörper aufweist, wobei das Paar benachbarter Einheitskörper einen ersten Einheitskörper und einen zweiten Einheitskörper aufweist,
die Mehrzahl von Verriegelungselementen mindestens ein Paar benachbarter Verriegelungselemente aufweist, wobei das Paar benachbarter Verriegelungselemente ein erstes Verriegelungselement, das an dem ersten Einheitskörper angebracht ist, und ein zweites Verriegelungselement, das an dem zweiten Einheitskörper angebracht ist, aufweist,
das erste und das zweite Verriegelungselement dazu ausgebildet sind, in einer neutralen Position nicht miteinander in Kontakt zu kommen, und
das erste und das zweite Verriegelungselement dazu ausgebildet sind, unter einer Last miteinander in Kontakt zu kommen,
**dadurch gekennzeichnet, dass** die Außenhaut (506, 606) dazu ausgebildet ist, die Mehrzahl von Einheitskörpern flexibel zu verbinden.

2. Flexible poröse Struktur nach Anspruch 1, wobei die Einheitskörper perforierte Scheiben sind.

3. Flexible poröse Struktur nach Anspruch 1 oder 2, wobei jedes der Mehrzahl von Verriegelungselementen eine geschichtete Struktur aufweist, und wobei Schichten benachbarter Verriegelungselemente zwischeinander angeordnet sind.

4. Flexible poröse Struktur nach einem der Ansprüche 1 bis 3, wobei die Außenhaut eine federartige Gestaltung aufweist.

5. Flexible poröse Struktur nach einem der Ansprüche 1 bis 3, wobei die Außenhaut eine Helix-Gestaltung oder eine Kreuzhelix-Gestaltung aufweist.

6. Flexible poröse Struktur nach Anspruch 4 oder 5, wobei die Außenhaut ferner zusätzliche Verriegelungselemente aufweist.

7. Flexible poröse Struktur nach einem der Ansprüche 1 bis 3, wobei die Außenhaut schlitzförmige Perforationen in einer versetzten Konfiguration aufweist.

8. Flexible poröse Struktur nach einem der Ansprüche 1 bis 7, wobei die Einheitskörper in einer Richtung gestapelt sind.

9. Flexible poröse Struktur nach einem der Ansprüche 1 bis 8, wobei die flexible poröse Struktur eine zylindrische Form aufweist.

10. Flexible poröse Struktur nach einem der Ansprüche 1 bis 8, wobei die flexible poröse Struktur einen polygonalen Querschnitt aufweist.

11. Flexible poröse Struktur nach Anspruch 10, wobei der polygonale Querschnitt ein Dreieck, Quadrat, Rechteck oder Sechseck aufweist.

12. Flexible poröse Struktur (900) zur Einpflanzung in einen Knochen, aufweisend eine plattenartige Struktur, wobei die plattenartige Struktur eine Mehrzahl von flexiblen porösen Strukturen nach Anspruch 10 oder 11 aufweist.

13. Flexible poröse Struktur (1000) zur Einpflanzung in einen Knochen, aufweisend ein Volumen, wobei das Volumen eine Mehrzahl von flexiblen porösen Strukturen nach Anspruch 10 oder 11 aufweist.

14. Implantat,
wobei das Implantat ein knochendefektfüllendes Implantat für einen länglichen Knochen ist, wie beispielsweise ein Radius, eine Ulna, ein Humerus, ein Femur, eine Tibia, eine Fibula, eine Rippe, eine Clavicula, eine Mandibula, eine Phalange, ein Metacarpus oder ein Metatarsus, und das knochendefektfüllende Implantat eine flexible poröse Struktur nach einem der Ansprüche 1 bis 9 aufweist,
wobei das Implantat eine flexible poröse Struktur nach Anspruch 12 aufweist, wobei die plattenartige Struktur eine knochenkontaktierende Oberfläche des Implantats bedeckt,
wobei das Implantat eine flexible poröse Struktur nach Anspruch 12 aufweist, wobei das Implantat ein plattenartiges Implantat ist, und wobei die plattenartige Struktur einen Körper des Implantats bildet, oder
wobei das Implantat eine flexible poröse Struktur nach Anspruch 13 aufweist, wobei das Volumen zumindest einen Teil eines Körpers des Implantats bildet.

## Revendications

1. Une structure poreuse flexible (500, 600) pour implantation dans un os, la structure comprenant :
une pluralité de corps unitaires (502, 602) ;
une peau extérieure (506, 606) ; et
une pluralité d'éléments d'emboîtement (504, 604),
dans laquelle :
la pluralité de corps unitaires comprend au moins une paire de corps unitaires adjacents, ladite paire de corps unitaires adjacents comprenant un premier corps unitaire et un second corps unitaire ;
la pluralité d'éléments d'emboîtement comprend au moins une paire d'éléments d'emboîtement adjacents, ladite paire d'éléments d'emboîtement adjacents comprenant un premier élément d'emboîtement fixé au premier corps unitaire, et un second élément d'emboîtement fixé au second corps unitaire ;
les premier et second éléments d'emboîtement sont configurés pour ne pas venir en contact l'un avec l'autre dans une position neutre ; et
les premier et second éléments d'emboîtement sont configurés pour entrer en contact l'un avec l'autre sous une charge,
**caractérisée en ce que** la peau extérieure (506, 606) est configurée pour relier de manière flexible la pluralité de corps unitaires.

2. La structure poreuse flexible selon la revendication 1, dans laquelle les corps unitaires sont des disques perforés.

3. La structure poreuse flexible selon la revendication 1 ou 2, dans laquelle chacun de la pluralité d'éléments d'emboîtement comprend une structure en couches, et dans laquelle des couches d'éléments d'emboîtement adjacents sont interposées les unes entre les autres.

4. La structure poreuse flexible selon l'une quelconque des revendications 1 à 3, dans laquelle la peau extérieure comprend une conception de type ressort.

5. La structure poreuse flexible selon l'une quelconque des revendications 1 à 3, dans laquelle la peau extérieure comprend une conception en hélice ou une conception en hélices croisées.

6. La structure poreuse flexible selon la revendication 4 ou 5, dans laquelle la peau extérieure comprend en outre des éléments d'emboîtement supplémentaires.

7. La structure poreuse flexible selon l'une quelconque des revendications 1 à 3, dans laquelle la peau extérieure comprend des perforations en forme de fente dans une configuration décalée.

8. La structure poreuse flexible selon l'une quelconque des revendications 1 à 7, dans laquelle les corps unitaires sont empilés dans une direction.

9. La structure poreuse flexible selon l'une quelconque des revendications 1 à 8, dans laquelle la structure poreuse flexible comprend une forme cylindrique.

10. La structure poreuse flexible selon l'une quelconque des revendications 1 à 8, dans laquelle la structure poreuse flexible comprend une section transversale polygonale.

11. La structure poreuse flexible selon la revendication 10, dans laquelle la section transversale polygonale comprend un triangle, un carré, un rectangle ou un hexagone.

12. Une structure poreuse flexible (900) pour implantation dans un os, comprenant une structure de type plaque, ladite structure de type plaque comprenant une pluralité de structures poreuses flexibles selon la revendication 10 ou 11.

13. Une structure poreuse flexible (1000) pour implantation dans un os, comprenant un volume, ledit volume comprenant une pluralité de structures poreuses flexibles selon la revendication 10 ou 11.

14. Un implant,
dans lequel l'implant est un implant de comblement de défaut osseux pour un os allongé, tel qu'un radius, une ulna, un humérus, un fémur, un tibia, une fibula, une côte, une clavicule, une mandibule, une phalange, un métacarpien ou un métatarsien, et ledit implant de comblement de défaut osseux comprend une structure poreuse flexible selon l'une quelconque des revendications 1-9 ;
dans lequel l'implant comprend une structure poreuse flexible selon la revendication 12, dans lequel la structure de type plaque couvre une surface en contact avec l'os de l'implant ;
dans lequel l'implant comprend une structure poreuse flexible selon la revendication 12, dans lequel l'implant est un implant de type plaque, et dans lequel la structure de type plaque forme un corps de l'implant ; ou
dans lequel l'implant comprend une structure poreuse flexible selon la revendication 13, dans lequel le volume forme au moins une partie d'un corps de l'implant.
